# DEMANDE DE BREVET EUROPEEN

(11) **EP 3 783 617 A1**
(43) Date de publication de la demande: **24.02.2021**
(21) Numéro de dépôt: 20183545.1
(22) Date de dépôt: 01.07.2020
(51) Int. Cl.: G16H 20/17

(54) **SYSTÈME DE SURVEILLANCE A DISTANCE D'UN PATIENT ATTEINT DE LA MALADIE DE PARKINSON**

(30) Priorité: 19.08.2019 FR 1909262
(71) Demandeur: Pharma Dom, 94250 Gentilly (FR); L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Inventeur: KHUDYAKOV, Andrey, 94250 Gentilly (FR)
(74) Mandataire: Air Liquide

(57) **Abrégé**

L'invention concerne un système de télésurveillance (100) d'un patient (P) atteint de la maladie de Parkinson comprenant une pompe de perfusion (1) comprenant des moyens de pilotage pour délivrer un médicament antiparkinsonien en fonction d'une grandeur d'un paramètre de fonctionnement réglé au niveau de la pompe de perfusion (1), en particulier le débit de perfusion; des moyens de transmission de signal (101) pour recueillir et transmettre à distance (102) un signal représentatif de ladite grandeur; des moyens informatiques de traitement de données (103) pour recevoir et pour fournir ledit signal à des moyens d'affichage (104) pour afficher la grandeur dudit paramètre de fonctionnement; et des moyens de saisie (105) permettant à un utilisateur de modifier la grandeur affichée par les moyens d'affichage (104).

## Description

L'invention concerne un système de suivi à distance, i.e. de télésuivi et/ou de télésurveillance, d'un patient atteint de la maladie de Parkinson, en particulier d'un patient parkinsonien traité à son domicile par administration d'un médicament antiparkinsonien, de préférence dopaminergique (ATC N04B), au moyen d'une pompe de perfusion contrôlée à distance.

La maladie de Parkinson est une maladie neurologique chronique se caractérisant par une destruction des neurones à dopamine présentant dans le cerveau des individus atteints de cette maladie, appelés patients parkinsoniens.

Les neurones à dopamine sont impliqués dans le contrôle des mouvements du corps et leur destruction engendre alors différents symptômes chez ces patients parkinsoniens, comme des tremblements, des raideurs musculaires, une lenteur de mouvement, des mouvements involontaires plus ou moins importants, appelés dyskinésies,une rigidité musculaire, une instabilité posturale, des blocages (ou freezing Phase Off en anglais)...

Pour traiter cette maladie, on administre aux patients différents médicaments antiparkinsoniens, notamment dopaminergiques, permettant de stimuler les neurones à dopamine, telle la L-Dopa. Toutefois, il a été remarqué que ce type de médicament, en particulier la L-Dopa, présente chez certains patients parkinsoniens des fluctuations d'efficacité.

Dès lors, chez les patients parkinsoniens présentant de telles fluctuations d'efficacité et/ou dans le cas de dyskinésies sévères, il est préconisé d'utiliser d'autres médicaments antiparkinsoniens, telle l'apomorphine qui est un stimulateur des récepteurs dopaminergiques postsynaptiques exerçant alors une action antiparkinsonienne.

L'apomorphine est généralement administrée automatiquement au patient au moyen d'une pompe à perfusion délivrant des doses d'apomorphine durant la journée au patient, via un cathéter implanté en sous-cutané chez le patient.

Le patient peut aussi de lui-même, activer la pompe pour qu'elle délivre une dose supplémentaire d'apomorphine, appelée bolus, par exemple en cas de tremblement excessif.

De façon générale, il est indispensable, pour obtenir un traitement d'un patient parkinsonien pleinement efficace, de pouvoir délivrer au patient la bonne dose de médicament, en particulier de l'apomorphine, tout au long de la journée et surtout de pouvoir rectifier cette dose, c'est-à-dire la réduire ou l'augmenter, s'il est constaté que la dose ayant été fixée n'est pas (assez) efficace ou conduit à des effets négatifs chez le patient, notamment allergie, hallucinations, troubles du comportement, dyskinésies gênantes, somnolence toute la journée, nausées/vomissements sous dompéridone.

Or, les patients parkinsoniens ne sont généralement pas hospitalisés ou leur période d'hospitalisation est très courte pour définir la dose optimale pour toutes les situations de vies, c'est-à-dire que leur traitement à lieu à leur domicile, donc en l'absence de personnel soignant, i.e. médecin et/ou infirmière, pouvant s'assurer du bon déroulement du traitement et, le cas échéant, intervenir rapidement pour ajuster le traitement dispensé, par exemple la dose ou débit de médicament antiparkinsonien administré au patient ou un autre paramètre de fonctionnement de la pompe à perfusion.

Le problème qui se pose est dès lors de pouvoir détecter rapidement un traitement par perfusion d'un médicament contre la maladie de Parkinson, c'est-à-dire un médicament antiparkinsonien, telle de l'apomorphine, de la duodopa® ou autre, qui est non ou insuffisamment efficace pour traiter un patient parkinsonien qui se trouve hors hôpital, en particulier à son domicile, et de pouvoir si possible intervenir rapidement i.e. rétroagir, à distance afin d'opérer un ou des ajustements des réglages de la pompe délivrant le médicament au patient considéré.

La solution de l'invention concerne un système de suivi à distance, i.e. un dispositif médical de télésuivi et/ou de télésurveillance, d'au moins un patient atteint de la maladie de Parkinson, c'est-à-dire un patient parkinsonien, comprenant :
- une pompe de perfusion comprenant des moyens de pilotage pour délivrer un médicament en fonction d'au moins une grandeur d'au moins un paramètre de fonctionnement réglé au niveau de la pompe de perfusion,
- des moyens de transmission de signal coopérant avec la pompe de perfusion et configurés pour recueillir et transmettre à distance au moins un signal représentatif de ladite grandeur du paramètre de fonctionnement de la pompe de perfusion considéré,
- des moyens informatiques de traitement de données configurés pour recevoir ledit au moins un signal transmis par des moyens de transmission de signal et pour fournir ledit au moins un signal à des moyens d'affichage,
- des moyens d'affichage configurés pour afficher la grandeur dudit paramètre de fonctionnement correspondant audit signal fourni par les moyens informatiques de traitement de données, et
- des moyens de saisie permettant à un utilisateur de modifier la grandeur dudit paramètre de fonctionnement affichée par les moyens d'affichage,
caractérisé en ce que :
- la pompe de perfusion comprend au moins un réservoir contenant un médicament antiparkinsonien sous forme liquide, ledit au moins un réservoir étant amovible, c'est-à-dire détachable de la pompe, et ayant une contenance inférieure ou égale à 100 ml,
- une grandeur de paramètre de fonctionnement réglé au niveau de la pompe de perfusion est une valeur de débit comprise entre 0 et 8 ml/h ou entre 0 et 40 mg/h, et
- les moyens de pilotage commandent la fourniture de médicament provenant dudit au moins un réservoir en fonction de ladite valeur de débit.

Selon le mode de réalisation considéré, le système de suivi, i.e. de télésuivi et/ou de télésurveillance, de l'invention peut comprendre l'une ou plusieurs des caractéristiques suivantes :
- ledit au moins un réservoir contient un médicament antiparkinsonien dopaminergique, c'est-à-dire que le médicament antiparkinsonien contenu dans le réservoir est de type dopaminergique, i.e. un (ou des) agent dopaminergique.
- ledit au moins un réservoir contient un médicament antiparkinsonien choisi parmi l'apomorphine et la carbidopa, la levodopa, l'entacapone et/ou leurs mélanges, en particulier un mélange (i.e. une association ou combinaison) contenant carbidopa et levodopa, comme la Duodopa®, ou un mélange contenant carbidopa, levodopa et entacapone, comme le Lecigon®.
- la pompe de perfusion est configurée pour opérer une administration continue du médicament liquide à la valeur de débit de perfusion.
- la pompe de perfusion comprend un réservoir unique ou deux réservoirs juxtaposés.
- la pompe de perfusion comprend des moyens de pilotage pour délivrer un médicament à administration contrôlée, en particulier de l'apomorphine.
- la pompe de perfusion est configurée pour administrer le médicament antiparkinsonien par voie sous-cutanée ou par voie intestinale.
- les moyens d'affichage sont en outre configurés pour afficher la grandeur modifiée du paramètre de fonctionnement, en particulier une valeur de débit.
- les moyens informatiques de traitement de données sont configurés pour transmettre à distance aux moyens de transmission de signal au moins un signal-retour représentatif de la grandeur modifiée dudit paramètre de fonctionnement.
- les moyens de transmission de signal sont en outre configurés pour recevoir ledit au moins un signal-retour et transmettre ledit signal-retour à la pompe de perfusion.
- les moyens de transmission de données comprennent au moins un modem ou analogue.
- les moyens de pilotage de la pompe de perfusion sont configurés pour modifier (i.e. changer, ajuster ou analogue) la grandeur du paramètre de fonctionnement considéré, en particulier le débit de perfusion, en fonction dudit signal-retour reçu par la pompe de perfusion.
- les moyens de transmission de signal sont intégrés à la pompe de perfusion.
- de façon alternative, les moyens de transmission de signal sont compris dans un module de communication indépendant communiquant avec la pompe de perfusion, par exemple en mode Bluetooth.
- la grandeur du paramètre de fonctionnement réglée au niveau de la pompe est choisie parmi :
   - un horaire journalier de début de perfusion,
   - un horaire journalier de fin de perfusion,
   - un nombre de bolus journalier programmé,
   - un volume de bolus,
   - le débit de bolus,
   - l'intervalle de temps entre les bolus,
   - le réglage du débit dans les plages d'horaires (pré)définies,
   - le volume à perfuser et
   - la concentration du médicament à perfuser.
- le ou les autres paramètre réglés au niveau de la pompe sont notamment:
   - le type de réservoir et de l'unité,
   - les informations du patient, en particulier nom, prénom, date de naissance, nom du médicament administré....,
   - le mode de perfusion automatique (i.e. AUTO) ou manuel (MANUEL),
   - le ou les débits en mode AUTO et/ou MANUEL, et
   - la configuration des alarmes.
- les moyens informatiques de traitement de données comprennent au moins un serveur informatique.
- le serveur informatique comprend un ou plusieurs processeurs mettant en oeuvre un ou plusieurs algorithmes, logiciels, programmes ou analogue.
- le serveur informatique comprend un module de communication, notamment de type BLE, WIFI, Carte à puce 2G/3G/4G/5G ou autre.
- les moyens d'affichage comprennent un écran de visualisation, par exemple l'écran d'un (ou plusieurs) ordinateurs fixes ou portables, ou l'écran d'une tablette numérique ou un téléphone mobile multifonction (i.e. smartphone).
- les moyens d'affichage comprennent un écran de visualisation en couleurs ou monochrome (e.g. en noir et blanc).
- les moyens d'affichage comprennent un écran tactile.
- les moyens de saisie comprennent un clavier d'ordinateur ou un clavier numérique, tel un clavier digital de tablette numérique ou de téléphone mobile multifonction.
- le (ou les) réservoir est amovible, c'est-à-dire qu'il peut être détaché de la pompe, notamment lorsqu'il est vide afin de le remplacer ou de le remplir.
- la pompe comprend deux réservoirs juxtaposés, l'un des réservoirs contenant le médicament liquide et l'autre réservoir contenant le médicament liquide ou une solution additionnelle devant être mélangée au médicament liquide avant sont administration au patient, par exemple une solution tampon, par exemple une solution tampon permettant d'ajuster le pH du médicament liquide ou autre.
- le réservoir a une contenance inférieure ou égale à 90 ml, typiquement inférieure ou égale à 50 ml environ, par exemple 20 ml, 30 ml ou 50 ml.
- le (ou les) réservoir comprend un corps de réservoir creux, un piston mobile en translation dans ledit corps creux et un orifice de distribution de fluide, de préférence porté par un embout.
- le médicament liquide à injecter est stocké dans le réservoir creux d'au moins un réservoir.
- le corps de réservoir creux est de forme générale tubulaire, de préférence de section circulaire ou ovale.
- la pompe de perfusion comprend en outre un moyen d'actionnement, tel une tige ou un poussoir, venant coopérer avec le piston d'au moins un réservoir de manière à repousser le médicament liquide en direction de l'orifice de distribution de fluide, i.e. de sortie du médicament liquide.
- les moyens de pilotage de la pompe de perfusion commandent, directement ou indirectement, le déplacement dudit moyen d'actionnement de manière à fournir le médicament liquide au débit désiré.
- les moyens de pilotage de la pompe de perfusion commandent un déplacement en translation du moyen d'actionnement au sein du corps de réservoir.
- la pompe de perfusion comprend en outre un moteur électrique coopérant avec moyen d'actionnement et les moyens de pilotage de la pompe de perfusion commandent ledit moteur électrique pour opérer un déplacement dudit moyen d'actionnement de manière à fournir le médicament liquide au débit désiré.
- la pompe de perfusion comprend en outre des moyens de mémorisation, telle une mémoire configurée pour enregistrer (i.e. mémoriser) la ou les grandeurs désirées du ou des paramètres de fonctionnement considérés, en particulier une ou des valeurs de débit.
- la pompe de perfusion comprend en outre une source d'alimentation électrique, par exemples une ou plusieurs batteries électriques, de préférence une batterie rechargeable via un chargeur-secteur venant se raccorder à la pompe.
- la pompe de perfusion comprend en outre un boitier de pompe, c'est-à-dire une carcasse ou coque externe, portant un écran digital.
- la pompe de perfusion comprend en outre au moins un bouton de mise en marche et d'arrêt, i.e. « on/off ».
- la pompe de perfusion comprend en outre une trappe d'accès à un compartiment à batterie où est logée la (ou les) batterie(s) d'alimentation électrique.
- les moyens de pilotage commandent la fourniture de médicament provenant du réservoir en fonction de ladite au moins une grandeur dudit au moins un paramètre de fonctionnement réglé au niveau de la pompe de perfusion ou modifiée en fonction du signal-retour reçu par la pompe de perfusion, en particulier le débit de perfusion.
- les moyens de pilotage comprennent un microprocesseur embarqué dans la pompe de perfusion, par exemple porté par une carte électronique.

L'invention porte aussi sur une utilisation d'un système de télésuivi et/ou de télésurveillance selon l'invention pour opérer une surveillance/suivi et un traitement à distance d'un patient parkinsonien.

L'invention va maintenant être mieux comprise grâce à la description détaillée suivante, faite à titre illustratif mais non limitatif, en référence aux Figures annexées parmi lesquelles :
Fig. 1 schématise un système d'administration par perfusion d'un médicament antiparkinsonien chez un patient parkinsonien, et
Fig. 2 schématise un mode de réalisation d'un système de télésurveillance d'un patient parkinsonien selon l'invention.

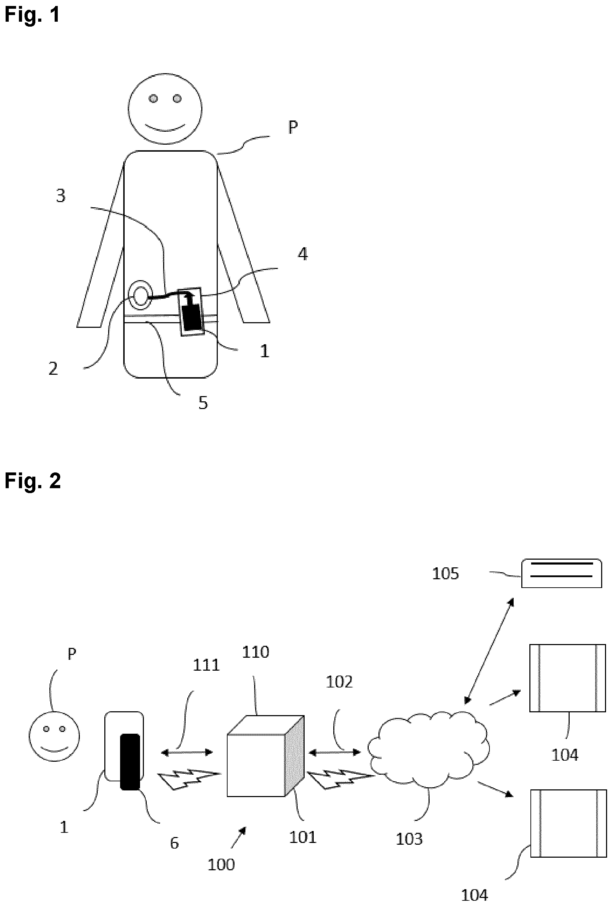

Fig. 1 schématise une administration par perfusion d'un médicament antiparkinsonien stimulateur des récepteurs dopaminergiques, à savoir ici de l'apomorphine par exemple, chez un patient parkinsonien. Toutefois, un autre médicament peut être utilisé en lieu et place de l'apomorphine, comme de la duodopa® (i.e. carbidopa/levodopa). On prend ci-après l'apomorphine comme exemple illustratif mais non limitatif.

Comme on le voit, l'administration de l'apomorphine (ou autre) se fait au moyen d'une pompe 1 de perfusion comprenant ici un réservoir 6 à piston relié à un cathéter 2 implanté en sous-cutané chez le patient P. Le réservoir 6 contenant l'apomorphine sous forme liquide a typiquement une contenant de quelques dizaines de ml (i.e. < 100 ml), par exemple 20, 30 ou 50 ml. Avantageusement, le réservoir 6 est amovible, c'est-à-dire qu'il peut être monté ou démonté du reste de la pompe 1, notamment pour le remplacer ou le remplir d'apomorphine lorsqu'il est vide.

Selon un autre mode de réalisation, la pompe 1 peut comprendre deux réservoirs agencés en parallèle fournissant chacun le médicament à injecter, telle de l'apomorphine, ou alors

Le cathéter 2 comprend une tubulure 3 raccordée à une sortie du réservoir de la pompe 1, servant à acheminer l'apomorphine jusqu'à une aiguille d'injection sous-cutanée qui permet de l'injecter au patient.

De préférence, un système adhésif ou analogue est prévu pour maintenir le cathéter en place sur le corps du patient. Il peut être fixé à différents endroits du corps, tels les flancs, les omoplates ou la zone péri-ombilicale.

Un tel ensemble de perfusion permet injecter l'apomorphine au patient P soit automatiquement et en continu pendant la journée, soit en bolus par activation de la pompe 1 par le patient lui-même, par exemple en cas de tremblement excessif nécessitant une délivrance d'une dose supplémentaire d'apomorphine.

En général, les injections d'apomorphine (ou autre médicament) se font pendant la journée, c'est-à-dire entre le lever et le coucher du patient P. Toutefois, dans certains cas, une ou des administrations d'apomorphine peuvent avoir lieu durant la nuit.

Les paramètres de fonctionnement de la pompe 1 sont réglés par un utilisateur, tel un médecin ou/et un infirmier, c'est-à-dire un professionnel de santé habilité, au niveau de la pompe et mémorisés au sein d'une mémoire intégrée à la pompe, en particulier une (ou des) valeur de débit de perfusion, un horaire journalier de début de perfusion et un horaire journalier de fin de perfusion, et éventuellement un nombre maximum de bolus journaliers autorisés et un volume de bolus,....

Par ailleurs, on prévoit aussi dans la pompe 1, des moyens de pilotage, tel un microprocesseur porté par une carte électronique, permettant de commander, directement ou indirectement, la fourniture d'apomorphine au débit désiré, par exemple en pilotant un moteur électrique commandant un déplacement translatif d'un moyen d'actionnement, tel un poussoir ou une tige, agissant sur le piston du réservoir dans lequel est contenue l'apomorphine de sorte de repousser ledit piston et donc aussi l'apomorphine (qui est poussée par le piston) en direction d'un orifice de distribution de fluide et alimentant ainsi en apomorphine la tubulure du cathéter 3.

La fourniture du médicament liquide au débit désiré est pilotée à distance par le système 100 de l'invention comme expliqué ci-après.

La pompe 1 à apomorphine est préférentiellement insérée dans un étui 4, une pochette, un petit sac ou analogue que le patient P peut par exemple être fixée à sa ceinture 5 (comme illustré), portée en bandoulière ou accrochée autour du cou.

D'une façon générale, le système de suivi 100 de l'invention décrit ci-après est compatible avec toutes les pompes 1 connectées de ce type ou analogue.

La Fig. 2 schématise un mode de réalisation d'un système de suivi 100 à distance, i.e. de télésurveillance ou télésuivi, d'un patient parkinsonien P selon l'invention.

Il comprend une pompe de perfusion 1 à réservoir amovible 6, tel que décrite ci-dessus, pour délivrer un médicament sous forme liquide, à savoir ici de l'apomorphine, en fonction d'au moins une grandeur d'au moins un paramètre de fonctionnement réglé au niveau de la pompe de perfusion 1, en particulier une valeur de débit de perfusion d'apomorphine typiquement compris entre 0 et 8 ml/h ou entre 0 et 40 mg/h (pour une concentration de 5 mg/ml).

La délivrance de l'apomorphine au débit désiré est contrôlée par des moyens de pilotage en mode auto ou manuel selon le paramétrage, tel un (ou des) microprocesseur porté par une carte électronique embarquée dans la pompe 1.

Sont aussi prévus des moyens de mémorisation, telle une carte mémoire ou analogue, intégrés à la pompe 1, par exemple portés par la carte électronique sur laquelle est agencé le microprocesseur. Ces moyens de mémorisation servent à enregistrer les paramètres de fonctionnement de la pompe 1 et les grandeurs/valeurs de ces paramètres, par exemple une valeur de débit de perfusion comprise entre 0 et 8 ml/h ou entre 0 et 40 mg/h, un horaire journalier de début de perfusion et/ou de fin de perfusion, un nombre de bolus journaliers autorisés, un volume de bolus... ou d'autres paramètres.

Dans le mode de réalisation du système 100 de l'invention illustré sur la Fig. 2, la pompe de perfusion 1 communique en mode Bluetooth 111 avec des moyens de transmission de signal 101 intégrés dans un module de communication 110 indépendant, tel un boitier connecté, communiquant avec la pompe de perfusion 1 et configurés pour recueillir et transmettre à distance 102 le (ou les) signal représentatif de la grandeur du paramètre de fonctionnement de la pompe de perfusion 1 considéré, à savoir ici une valeur de débit de perfusion.

Toutefois, selon un autre mode de réalisation (non montré), les moyens de transmission de signal 101 peuvent aussi être embarqués directement dans la pompe de perfusion 1.

Dit autrement, la pompe 1 comprend ici des moyens de télécommunication embarqués fonctionnant de préférence en Bluetooth, servant à émettre des signaux, c'est-à-dire données, informations, paramètres ou autres, vers les moyens de transmission de signal 101 intégrés dans le module de communication 110 indépendant, qui est lui-même communiquant, c'est-à-dire conçu pour recevoir puis retransmettre 102 le (ou les) signal représentatif de la grandeur du paramètre de fonctionnement de la pompe de perfusion 1 considéré, à savoir ici la valeur de débit de perfusion, à destination de moyens informatiques de traitement de données 103, tel un (ou plusieurs) serveur informatique situé à distance, c'est-à-dire sur un autre site. Les moyens informatiques de traitement de données 103 peuvent en outre comprendre un espace informatique virtuel, aussi appelé «cloud» ou «middleware».

De préférence, les moyens de transmission de signal 101 sont configurés pour transmettre à distance le ou les signaux, via le réseau internet ou un autre réseau de communication, tels les réseaux GSM, Wifi, Lora ou Sigfox. Par exemple, ils comprennent un modem de type GSM ou autre et une antenne émettrice.

Les moyens informatiques de traitement de données 103, c'est-à-dire le ou les serveurs et/ou un (ou des) un espace informatique virtuel (i.e. cloud), sont configurés pour recevoir (ou envoyer) le signal transmis par des moyens de transmission de signal 101, puis le fournir, après traitement informatique éventuel au moyen d'un (ou plusieurs) algorithme hébergé sur le (ou les) serveur, à des moyens d'affichage 104, à savoir un (ou plusieurs) écran digital, typiquement un écran de visualisation, par exemple l'écran ou moniteur d'un ordinateur, d'une tablette numérique ou d'un téléphone mobile multifonction (smartphone).

Les moyens d'affichage 104 permettent alors d'afficher la grandeur du paramètre de fonctionnement considéré, en particulier le débit de perfusion, correspondant au signal fourni par les moyens informatiques de traitement de données 103.

Un utilisateur, typiquement du personnel soignant, notamment médecin ou infirmièr(e), peut alors consulter la valeur du paramètre de fonctionnement considéré, en particulier le débit de perfusion, affiché sur l'écran, à savoir ici le débit de perfusion, et ainsi décider soit de ne rien modifier au fonctionnement de la pompe 1, soit au contraire décider de changer la valeur du paramètre considéré afin de la diminuer ou l'augmenter la titration (dose) de médicament par exemple, notamment de modifier ou ajuster le débit de perfusion.

Pour ce faire, il est prévu des moyens de saisie 105 permettant à l'utilisateur de modifier la grandeur du paramètre de fonctionnement affichée par les moyens d'affichage 104, e.g. le débit de perfusion. Ceux-ci comprennent par exemple un ordinateur fixe ou portable, un clavier numérique, une tablette numérique ou un téléphone mobile multifonction (smartphone) et une application web ou mobile qui est conçu pour accompagner le médecin ou autre professionnel de santé pour réaliser le contrôle des données et réajuster (prendre la décision) en fonction du besoin (télésurveillance / télésuivi).

Les moyens de saisie 105 coopèrent avec les moyens informatiques de traitement de données 103, c'est-à-dire le ou les serveurs, de sorte que la ou les modifications du ou des paramètres opérées, en particulier le débit de perfusion, soient prises en compte et/ou enregistrées par les moyens informatiques de traitement de données 103.

Une fois que l'utilisateur a modifié la grandeur du paramètre de fonctionnement affichée par les moyens d'affichage 104, en particulier le débit de perfusion, la grandeur modifiée, c'est-à-dire la nouvelle valeur de débit par exemple, est alors affichée par les moyens d'affichage 104.

Les moyens informatiques de traitement de données 103 retransmettent alors à distance 102, le (ou les) paramètre(s) considéré(s) aux moyens de transmission de signal 101 via un signal-retour représentatif de la grandeur modifiée du (ou des) paramètre de fonctionnement.

Les moyens de transmission de signal 101 réceptionnent ce signal-retour et le fournissent aux moyens de pilotage et/ou aux moyens de mémorisation de la pompe de perfusion 1.

Les moyens de pilotage sont configurés pour prendre en compte ce signal-retour, c'est-à-dire la (ou les) modifications opérées, pour modifier la grandeur du paramètre de fonctionnement considéré en fonction du signal-retour reçu par la pompe de perfusion 1 soit automatiquement, soit par une sous validation par un autre professionnel de santé lors dans la réception à distance, par exemple pour modifier le débit de perfusion réglé sur la pompe 1 et le remplacer par le nouveau débit de perfusion reçu, c'est-à-dire un débit plus élevé ou, à l'inverse, moins élevé que le débit initialement réglé sur la pompe 1.

D'une façon générale, le système de suivi à distance 100 de l'invention permet d'opérer aussi une collecte d'informations ou données, à savoir non seulement le ou les paramètres de fonctionnement provenant de la pompe 1, en particulier le débit de perfusion, mais aussi d'autres informations, données cliniques, paramètres ou autres provenant d'un (ou plusieurs) autre dispositif médical connecté et/ou des réponses à des questionnaires de santé ou autre, puis leur analyse et une restitution des données vers un ou des utilisateurs ou intervenants, tel le personnel soignant, notamment médecin, infirmier/ère ou autre à l'hôpital ou à domicile.

Par exemple, les données cliniques peuvent comprendre, pour chaque perfusion et chaque patient, la configuration de la pompe, la date et l'heure de début de perfusion et de fin de perfusion, le ou les débits de perfusion appliqués en cours d'une perfusion, la date et l'heure des bolus, la date, l'heure et le type des événements indésirables, la date et l'heure des alarmes médicaments, et plus généralement toute information du patient.

Le système 100 de l'invention permet donc de réaliser un suivi et/ou une surveillance en temps réel du traitement du patient parkinsonien.

De préférence, la pompe 1 du système 100 communique en Bluetooth Low Energy (BLE) pour économiser sa batterie électrique. La communication se fait habituellement une fois par jour avec des trames comportant une journée de données. Toutefois, un paramétrage de la fréquence d'envoi est possible pour s'adapter aux nécessités de suivi du patient, par exemple 2 fois par jour ou plus.

Bien entendu, en fonction de la criticité des données envoyées, un système de sécurisation de l'envoi, notamment un système de cryptage ou d'identification, peut être aussi prévu.

Les moyens de transmission de signal 101, en particulier le boîtier connecté 110, font office de passerelle de communication assurant une liaison et une transmission de signaux (i.e. données) entre la pompe de perfusion 1 présente au domicile du patient ou dans tout autre endroit (patient à l'hôpital, hors domicile...) et le ou les serveurs distants, e.g. un serveur dans le Cloud, formant tout ou partie des moyens informatiques de traitement de données 103.

Lorsqu'un boîtier connecté 110 est utilisé, il est préférentiellement automatique, autonome (i.e. branché au secteur), discret pour le patient, sans interface patient et éventuellement équipé d'une LED de bon fonctionnement.

La communication entre la passerelle, i.e. le boîtier connecté 110, et le serveur dans le Cloud 103 se fait habituellement en 2G-3G, 4G, Wifi ou autre, à une fréquence de transmission d'une fois par jour ou plus (possibilité de paramétrage), avec des trames comportant les données patient et techniques à envoyer.

Le serveur centralisé des moyens informatiques de traitement de données 103 assure la réception, le stockage, le traitement et l'affichage des données de patient, en particulier le (ou les) paramètres de fonctionnement, tel la (ou les) valeur du débit de perfusion réglé et/ou modifié/ajusté.

Le système 100 de suivi à distance de l'invention est particulièrement bien adapté au télésuivi et/ou à la télésurveillance d'un patient atteint de la maladie de Parkinson, en particulier d'un patient parkinsonien traité à son domicile par administration d'un médicament antiparkinsonien au moyen d'une pompe de perfusion, en particulier de l'apomorphine, d'une combinaison carbidopa/levodopa, tel que Duodopa®, ou d'une combinaison carbidopa/levodopa/entacapone.

## Revendications

1. Système de suivi à distance (100) d'au moins un patient (P) atteint de la maladie de Parkinson comprenant :
- une pompe de perfusion (1) comprenant des moyens de pilotage pour délivrer un médicament en fonction d'au moins une grandeur d'au moins un paramètre de fonctionnement réglé au niveau de la pompe de perfusion (1),
- des moyens de transmission de signal (101) coopérant avec la pompe de perfusion (1) et configurés pour recueillir et transmettre à distance (102) au moins un signal représentatif de ladite grandeur du paramètre de fonctionnement de la pompe de perfusion (1) considéré,
- des moyens informatiques de traitement de données (103) configurés pour recevoir ledit au moins un signal transmis par des moyens de transmission de signal (101) et pour fournir ledit au moins un signal à des moyens d'affichage (104),
- des moyens d'affichage (104) configurés pour afficher la grandeur dudit paramètre de fonctionnement correspondant audit signal fourni par les moyens informatiques de traitement de données (103), et
- des moyens de saisie (105) permettant à un utilisateur de modifier la grandeur dudit paramètre de fonctionnement affichée par les moyens d'affichage (104),
**caractérisé en ce que** :
- la pompe de perfusion comprend au moins un réservoir contenant un médicament antiparkinsonien sous forme liquide, le ou lesdits réservoirs étant amovibles et ayant une contenance inférieure ou égale à 100 ml,
- une grandeur de paramètre de fonctionnement réglé au niveau de la pompe de perfusion (1) est une valeur de débit comprise entre 0 et 8 ml/h ou entre 0 et 40 mg/h, et
- les moyens de pilotage commandent la fourniture de médicament antiparkinsonien provenant dudit au moins un réservoir (6) en fonction de ladite valeur de débit.

2. Système selon la revendication précédente, **caractérisé en ce que** ledit au moins un réservoir contient un médicament antiparkinsonien dopaminergique.

3. Système selon l'une des revendications précédentes, **caractérisé en ce que** ledit au moins un réservoir contient un médicament antiparkinsonien choisi parmi l'apomorphine, et la carbidopa, la levodopa, l'entacapone et leurs mélanges, en particulier un mélange contenant carbidopa et levodopa ou un mélange contenant carbidopa, levodopa et entacapone.

4. Système selon l'une des revendications précédentes, **caractérisé en ce que** les moyens d'affichage (104) sont en outre configurés pour afficher la grandeur modifiée du paramètre de fonctionnement.

5. Système selon l'une des revendications précédentes, **caractérisé en ce que** :
- les moyens informatiques de traitement de données (103) sont configurés pour transmettre à distance (102) aux moyens de transmission de signal (101) au moins un signal-retour représentatif de la grandeur modifiée dudit paramètre de fonctionnement,
- les moyens de transmission de signal (101) sont en outre configurés pour recevoir ledit au moins un signal-retour et transmettre ledit signal-retour à la pompe de perfusion (1), et
- les moyens de pilotage de la pompe de perfusion (1) sont configurés pour modifier la grandeur du paramètre de fonctionnement considéré en fonction dudit signal-retour reçu par la pompe de perfusion (1).

6. Système selon l'une des revendications précédentes, **caractérisé en ce que** les moyens de transmission de signal (101) sont intégrés à la pompe de perfusion (1) ou les moyens de transmission de signal (101) sont compris dans un module de communication (110) indépendant communiquant avec la pompe de perfusion (1), de préférence en mode Bluetooth (111).

7. Système selon l'une des revendications précédentes, **caractérisé en ce qu'**une autre grandeur du paramètre de fonctionnement réglée au niveau de la pompe (1) comprend en outre :
- un horaire journalier de début de perfusion,
- un horaire journalier de fin de perfusion,
- un nombre de bolus journaliers programmé,
- un volume de bolus,
- le débit de bolus,
- l'intervalle de temps entre les bolus,
- le réglage du débit dans les plages d'horaires définies,
- le volume à perfuser et
- la concentration du médicament à perfuser.

8. Système selon l'une des revendications précédentes, **caractérisé en ce que** la pompe de perfusion (1) comprend en outre un moyen d'actionnement et un moteur électrique coopérant avec le moyen d'actionnement, et les moyens de pilotage de la pompe de perfusion sont configurés pour commander, directement ou indirectement, le déplacement dudit moyen d'actionnement de manière à fournir le médicament liquide au débit désiré.

9. Système selon l'une des revendications précédentes, **caractérisé en ce que** les moyens de pilotage de la pompe de perfusion (1) sont configurés pour commander le moteur électrique pour opérer un déplacement du moyen d'actionnement de manière à fournir le médicament liquide au débit désiré.

10. Système selon l'une des revendications précédentes, **caractérisé en ce que** les moyens informatiques de traitement de données (103) comprennent au moins un serveur informatique distant.

11. Système selon l'une des revendications précédentes, **caractérisé en ce que** les moyens d'affichage (104) comprennent au moins un écran de visualisation.

12. Système selon l'une des revendications précédentes, **caractérisé en ce que** les moyens de saisie (105) comprennent un clavier d'ordinateur ou un clavier numérique, en particulier un clavier digital de tablette numérique ou de téléphone mobile multifonction.

13. Système selon l'une des revendications précédentes, **caractérisé en ce que** les moyens de pilotage commandent la fourniture de médicament provenant du réservoir (6) en fonction de ladite au moins une grandeur dudit au moins un paramètre de fonctionnement réglée au niveau de la pompe de perfusion (1) ou modifiée en fonction du signal-retour reçu par la pompe de perfusion (1).

14. Système selon l'une des revendications précédentes, **caractérisé en ce que** la pompe de perfusion comprend un réservoir unique ou deux réservoirs juxtaposés.

15. Système selon l'une des revendications précédentes, **caractérisé en ce que** la pompe de perfusion (1) est configurée pour administrer le médicament antiparkinsonien par voie sous-cutanée ou par voie intestinale.
